# EUROPEAN PATENT SPECIFICATION

(11) **EP 4 069 325 B1**
(45) Date of publication and mention of the grant of the patent: **05.03.2025**
(21) Application number: 20800264.2
(22) Date of filing: 22.10.2020
(51) Int. Cl.: A61L 27/18, A61L 27/20, A61L 27/24, A61L 27/36, A61L 27/52

(54) **A DBM SCAFFOLD PRODUCT AND A METHOD OF MANUFACTURING THE SAME**
DBM-GERÜSTPRODUKT UND VERFAHREN ZUM HERSTELLEN DESSELBEN
PRODUIT D'ÉCHAFAUDAGE DBM ET SON PROCÉDÉ DE FABRICATION

(30) Priority: 04.12.2019 GB 201917673
(43) Date of publication of application: 12.10.2022
(73) Proprietor: Veterinary Tissue Bank Ltd., Chirk, Wrexham Clwyd LL14 5NS (GB)
(72) Inventor: MYINT, Peter, Wrexham Clwyd LL14 5NS (GB); INNES, John Francis, Wrexham Clwyd LL14 5NS (GB)
(74) Representative: Johnstone, Edward Ian
(86) International application number: PCT/GB2020/052670
(87) International publication number: WO 2021/111101

(56) References cited:
- EP-A1- 3 275 470
- EP-B1- 3 275 470
- WO-A2-02/069818
- US-A1- 2004 249 463

## Description

The present invention relates to a demineralised bone matrix (DBM) scaffold product, for use particularly in orthopaedic procedures.

DBM is used in various orthopaedic procedures and dental/maxillofacial reconstructive surgery where accelerated fusion of bone is desired. Unlike conventional bone grafting materials which act as an osteoconductive scaffold to fill the bone defects, DBM contains growth factors such as bone morphogenic proteins (BMPs) to induce new bone formation by transforming stem cells to bone cells, and as such is both osteoconductive and osteoinductive.

DBM is formed from cortical bone, and is usually taken from the outer hard part of a donor long bones such as the femur or tibia. This material is hard and unlike the spongy bone material which is found inside the bone. Therefore, traditionally the sourced bone material is milled into small particles in the range of about 125um to 1mm in size, and is then subjected to a demineralisation process by being soaked in a mild acid solution, normally 0.5M or 0.6M hydrochloric acid. The demineralisation process removes minerals, mainly calcium, from the bone. This allows the growth factors which reside in the remaining collagen matrix of the bone to express their biological function when implanted into the body.

DBM products are then prepared for clinical use. The product can be in the form of the powder itself, but this can be difficult to handle being powder, and because it needs to be rehydrated before being implanted into the patient. Therefore, it is known to mix the powder with a biocompatible viscous solution so it can be injected, or formed into a mouldable putty. The viscous compounds used include glycerol, hyaluronic acid, gelatin, collagen, poloxamer and so on. The majority of these mixtures are made available in syringes for easy application.

Alternatively, DBM can be made from elongate bone fibres rather than particles. In particular, GB1711130.3 in the name of the Applicant discloses an apparatus and method for obtaining such bone fibres from donor bones by moving a cutting head reciprocally over the surface of a rigid bone secured in a vice. Bone fibres are believed to provide improved bone healing properties because they are elongate and mesh together to form a complex scaffold, which allows host cells to migrate into the implanted graft instead of having to "jump" from one particle to another. Bone fibres also tend to stay in situ in the bone defect compared to the particles which can easily be washed away by the body fluid. A mesh of bone fibres is also easy to handle as it can be picked up by hand or with forceps.

However, compared to the powder based DBM products, such as DBM power mixed with a viscous solution, the bone fibre based DBM products don't have the same advantageous smooth texture and tackiness which make the powder based DBM products easy to manipulate and implant. In addition, the bone fibres can fail to mesh together, which means that the product can fall apart or individual fibres come loose. If the bone fibres are too long they are less flexible, but if they are too short they become less entangled. This lack of consistency is not ideal for clinical use, and there is therefore a need to better interconnect the bone fibres together, so the DBM product is formed into a single cohesive mass.

US 2017/0312079 to Schlachter et al. discloses a bone material comprising strips of bone fibre which are mechanically entangled together to form a coherent mass. A number of methods are disclosed for achieving this mechanical entanglement, including needle punching with barbed needles, using air or water jets, or by applying ultrasonic waves. However, each of these methods results in an entirely random entanglement of the bone fibres. Each method involves applying forces to the subject bone fibres in order to agitate them such that they entangle together, or actually physically connect with one another. In fibrous product manufacturing terms these methods are essentially nothing more than felting.

The methods disclosed in Schlachter suffer from a number of drawbacks. Firstly, due to the randomised nature of the resulting DBM product, it has inconsistent performance characteristics. Some areas may comprise an effective bone formation scaffold, while other areas may not. Secondly, the resulting DBM product can still fall apart, or individual fibres come loose. Finally, the agitation methods disclosed are complex and difficult to apply in practice.

US 2004/249463 to Bindseil et al. discloses load bearing implants for use in fusion of adjacent bony structures. The implants disclosed in Bindseil are formed by one or more sheets comprising bone pieces, at least some of which may be at least partially demineralised. In some embodiments the individual sheets are formed by weaving elongate bone pieces together. The finished product is then assembled by folding and/or stacking one or more of the sheets together to form a solid structure capable of acting as a load bearing cushion.

The present invention is intended to overcome disadvantages of known DBM products.

The present invention is defined by the independent claims, while the dependent claims concern the preferred embodiments. Any "embodiment" or "example" which is disclosed in the description but is not covered by the claims should be considered as presented for illustrative purpose only. This applies for example to the DBM scaffold products comprising a plurality of elongate demineralised bone fibres woven together in a regular and repeating weave pattern, and methods of manufacturing thereof.

Therefore, according to a first aspect of the present invention a demineralized bone matrix (DBM) scaffold product comprises a plurality of elongate demineralised bone fibres knitted together in a regular and repeating knit pattern.

Thus, the present invention addresses the issues associated with the prior art bone fibre based DBM products by mechanically interconnecting the elongate bone fibres together in a uniform way. This results in regular performance across the area of the product because the bone fibres are arranged in the same manner in relation to one another throughout. It also prevents the product from easily deforming or from individual fibres coming loose. It also means that certain two dimensional shapes and three dimensional forms can be created by means of the mechanical interconnection method used, which shapes might find particular application in clinical use. The resulting products are easy to handle, and if flat they can be rolled or folded into more complex shapes depending on the clinical requirement.

Depending on the shape and size of the DBM scaffold product it can be used in various surgical procedures such as spinal fusion, bone augmentation, guided tissue regeneration in dental procedures and so on. The product would find particular application in posterolateral spinal fusion where the fibres could be specifically orientated across 2 or 3 lateral spinal processes due to their regular and repeating pattern. This is something which is not possible with any known DBM products in a neat and organised way.

The bone fibres can be procured in any known way from donor bones, but preferably they are obtained using the apparatus and method described in GB1711130.3 in the name of the Applicant. The principal reason for this is that the demineralised bone fibres produced using this apparatus and method are relatively resilient and can be readily manipulated in order to interconnect them. They are also substantially equal in length, width and depth, as they are produced using the same reciprocally activated bone rake, which has a number of identical cutting teeth. Other bone fibres produced using other known methods may be too rigid or brittle to be readily interconnected, or they may be irregular in shape and size.

A knitted product is one in which rows of material strands are looped and entwined together into stitches, with each loop of one row passing around the base of a loop of the row above. There are many known knitting techniques, and any can be employed with the present invention. For example, the bone fibres can be knitted together in a single crochet, into multiple knitted rows, or into whole sheets. The knitting can be performed by hand, or by a knitting machine. If performed by hand tools can be used, such as knitting needles, crochet needles or fine tip forceps. Once again, how tightly the strands of bone fibre are knitted together can be a matter for the person skilled in the art. The more closely bound the fibres are the more dense the final product will be, while the less closely bound the fibres are the more loose and maliable the final product will be. It will also be appreciated that the final knitted product can be any size and shape, provided that there is a regular and repeating knitting pattern. This means knitted DBM scaffold products can be made which are suitable in shape and size for particular clinical procedures. In particular, it is possible to form three dimensional knitted products, as discussed in more detail below.

While it is possible to form knitted DBM scaffold products as described above using single bone fibres, the utility of this approach is limited because bone fibres are relatively short in length. They are usually no longer than 15 to 20cms, depending on the source bone. Therefore, where individual bone fibres are used the resulting DBM scaffold product will be relatively small in size. There may still be valid application for such DBM scaffold products, depending on the clinical procedure in question. Therefore in one version of the invention the elongate demineralised bone fibres can each comprise a single filament taken from a donor bone.

However, in a preferred construction the elongate demineralised bone fibres can each comprise a composite strip comprising a plurality of filaments taken from donor bones which are connected together by an adhesive. With this construction the individual filaments of bone taken from donor bones are connected together to form any length of composite strips to form the strands for knitting.

The adhesive can comprise a liquid or gel tissue adhesive comprising one or more of a fibrin glue, cyanoacrylate based product, polyethylene glycol-PE-sealant or glutaraldehyde albumin-derived product. Such adhesives are biocompatible and normally used for closing lacerations and incisions. They are strong enough to connect bone fibres together sufficiently for composite strips to be made which can be knitted together as described above. The adhesive can further comprise a haemostatic component. These are currently used to stop bleeding and would assist in bonding the two bone fibres together.

The individual filaments can comprise flat sides, and they can be arranged with end portions thereof overlapping, and with the adhesive located between the overlapped end portions. This leads to a strong and effective bond. The individual filaments could also be connected together end face to end face, but this would not be as strong or effective because the end faces are small in area.

The invention is most likely to be carried out with composite strips as described above, but it could also be carried out with wound composite strips made up of multiple composite strips like those described above which are wound together to form a basic kind of yarn or rope-like structure. This might be preferred to increase the density of the final product, or to increase the strength of the composite strips forming the strands for knitting.

As explained above, the DBM scaffold product can be formed into any shape, but in one version it is formed into a sheet. In another version of the invention it is formed into a three-dimensional shape defining an inner area. In particular a basic bag or pouch formation can be created using any known knitting technique, which can be used to contain autograft or allograft bone chips therein. This may find particularly beneficial application in certain clinical procedures, where different kinds of bone material can be advantageously located in different areas. For example, the mechanically interconnected DBM bone fibres can be adjacent the surfaces of bone to be treated, while the larger bone chips inside the bag or pouch are held in the area between the surfaces to be treated.

In posterolateral spinal fusion surgery for instance where there are bone grafts on both sides of the spine it is known to use bags of bone chips, which are placed specifically to fill the bone defects. In the known products the bags are simply made of biodegradable resorbable mesh. An example is the Magnifuse (RTM) product produced by Medtronic. In the present invention these bags are replaced with the knitted DBM scaffold bags as described above, which would significantly improve performance.

In one version of the invention the DBM scaffold product can comprise a plurality of elongate synthetic fibres mechanically interconnected with the plurality of elongate demineralised bone fibres in a regular and repeating pattern. Such synthetic fibres could be polylactic acid, polyglycolic acid, collagen, chitosan, and so on. These could be used in order to make a composite product which has greater strength than otherwise. Such synthetic fibres could form alternate strands with composite strips of DBM bone fibres in the knitted product.

The use of synthetic fibres in this way allows for the biodegradation rate of the DBM scaffold product to be specifically adjusted. In particular, the DBM scaffold products could contain different pre-determined ratios of bone fibres to synthetic fibres, for example 1:1, 2:1, 3:1 and so on, depending on the desired biological and physical properties that would be achieved. This ratio would be simple to achieve by replacing the required number of composite strips of DBM bone fibres with synthetic fibre strips.

Alternatively, the synthetic fibres could be wound together with the DBM bone fibres to produce wound composite strips made up of both which are then used as the strands in the knitting process. This could be beneficial because the synthetic fibres can be produced in any length without the need for any connections, and their consistent strength along their length could help to support the connections between the individual filaments of bone in the composite strip of bone fibres with which they are wound.

The **DBM** scaffold product of the invention can be used without any of the known additional viscous compounds usually used with **DBM** products. However in one version of the invention a gel or viscous liquid can be applied to the plurality of elongate demineralised bone fibres. Adding such a gel or viscous liquid to the **DBM** scaffold product can make it more cohesive as a scaffold and it can give it superior handling characteristics. The gel or viscous liquid used can comprise one or more of glycerol, collagen, gelatin, hyaluronic acid, lecithin, calcium sulphate, poloxamer, calcium phosphate or carboxymethyl cellulose (CMC). These are known materials to use with **DBM** products.

A second aspect of the present invention provides a method of manufacturing a DBM scaffold product comprising a plurality of elongate demineralised bone fibres comprising the step of knitting said plurality of elongate demineralised bone fibres together with one another in a regular and repeating knit pattern.

As mentioned above, this manufacturing method can be carried out with individual filaments of bone. However, preferably prior to the step of knitting the plurality of elongate demineralised bone fibres together with one another in a regular and repeating knit pattern the method can comprise the step of forming each of the elongate demineralised bone fibers by connecting a plurality of filaments taken from donor bones together with adhesive to form a composite strip.

The filaments can comprise flat sides, and the step of forming each of the elongate demineralised bone fibres as a composite strip can comprise arranging adjacent filaments with end portions thereof overlapping, and locating the adhesive between the overlapped end portions.

The step of knitting the plurality of elongate demineralised bone fibres together with one another in a regular and repeating knit pattern can comprise forming the product as a sheet. Alternatively, that step can comprise forming the product as a three-dimensional shape defining an inner area.

The method can comprise the further step of placing the DBM scaffold product into a mould of a pre-determined shape and size and applying a pressure thereto in order to form the DBM scaffold product into a pre-determined shape. In particular, single DBM scaffold sheets can be press fitted together to make more dense sheets. These may find particular application in supporting bone fractures by being wrapped around the fracture site. The DBM scaffold product like this will retain its matrix structure of bone fibres in vivo, unlike current DBM flex products in which the bone fibres are randomly orientated.

Furthermore, this step of the method could involve pressing the DBM scaffold product into other known implant structures, such as boat like structures which can carry bone chips therein, and which are commonly inserted into the spine in spinal fusion surgery. Finally, the DBM scaffold product can be formed into dense elongate strips for use in other clinical procedures.

The method of the second aspect of the present invention can also include the step of mechanically interconnecting the bone fibres with elongate synthetic fibres in a regular and repeating pattern in order to create a composite DBM scaffold product comprising natural and synthetic fibres. As explained above, this can be done to improve strength, and to control the biodegradation rate of the product. The DBM scaffold product produced by the method could contain different pre-determined ratios of bone fibres to synthetic fibres, for example 1:1, 2:1, 3:1 and so on, depending on the desired biological and physical properties that would be achieved.

The method can also include the step of adding a gel or viscous liquid to the plurality of elongate demineralised bone fibres. Adding such a gel or viscous liquid to the DBM scaffold product can make it more cohesive as a scaffold and it can give it superior handling characteristics.

Fourteen embodiments of the invention will now be described by way of example and with reference to the accompanying drawings, in which:
Figure 1 is a front view of a first DBM scaffold product not according to the claimed invention;
Figure 2 is a front view of a second DBM scaffold product according to the first aspect of the present invention;
Figure 3 is a side view of a section of a composite DBM fibre strip used in manufacturing a third DBM scaffold product according to the first aspect of the present invention;
Figure 4 is a photograph of a fourth DBM scaffold product according to the first aspect of the present invention;
Figure 5 is a photograph of a fifth DBM scaffold product according to the first aspect of the present invention;
Figure 6 is a photograph of a sixth DBM scaffold product according to the first aspect of the present invention;
Figure 7 is a front view of a seventh DBM scaffold product not according to the claimed invention;
Figure 8 is a front view of an eighth DBM scaffold product not according to the claimed invention;
Figure 9 is a perspective front view of a ninth DBM scaffold product according to the first aspect of the present invention;
Figure 10 is a perspective view of a tenth DBM scaffold product according to the first aspect of the present invention;
Figure 11 is a sectional front view of an eleventh DBM scaffold product not according to the claimed invention;
Figure 12 is a side view of wound composite DBM fibre strip for use in manufacturing a twelfth DBM scaffold product according to the first aspect of the present invention;
Figure 13 is a side view of wound composite DBM fibre strip for use in manufacturing a thirteenth DBM scaffold product according to the first aspect of the present invention;
Figure 14 is a sectional front view of a fourteenth DBM scaffold product not according to the claimed invention; and
Figure 15 is a diagrammatic view of a method of manufacturing a DBM scaffold product not according to the claimed invention.

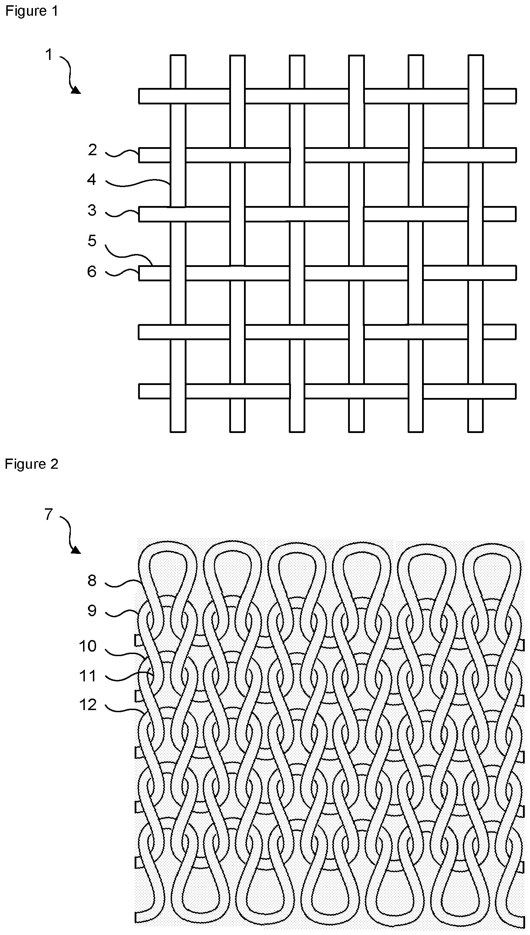

For the different DBM scaffold products described here below, only the DBM scaffold products comprising knitted demineralised bone fibres are according to the claimed invention. The DBM scaffolds comprising woven demineralised bone fibres are to be considered as reference-embodiments, not according to the claimed invention. As shown in Figure 1 a first DBM scaffold product 1 comprises a plurality of elongate demineralised bone fibres 2 mechanically interconnected with one another in a regular and repeating pattern.

It comprises 12 individual filaments 3, each of which is a single bone fibre 2 taken from a donor bone according to the method defined in GB1711130.3 in the name of the Applicant. As a result of the use of this method the bone fibres 2 are substantially equal in length, width and depth.

The bone fibres 2 are woven together in a regular and repeating pattern. Namely, an array of six parallel warp strands 4 are intertwined with an array of six parallel weft strands 5. Each of the warp strands 4 and each of the weft strands 5 passes reciprocally between the warp strands 4 or weft strands 5 normal to it. As such, the first DBM scaffold product 1 is a basic square DBM sheet with a mechanical interconnection pattern which is regular, and which is repeated nine times.

The first DBM scaffold product 1 has the bone fibres 2 arranged in a relatively spaced relationship with each other, whereas in practice this spacing may be much narrower in order to improve the structural integrity. Also, the first DBM scaffold product 1 does not feature any mechanism to secure the bone fibres 2 along the edges 6. Given its small size it would likely fall apart if manipulated. However, in practice DBM scaffold products would be manufactured which were far larger in size, and which may also feature a seam or other mechanism along the edges to maintain structural integrity. For example, with a woven pattern like that shown in Figure 1 ends of the bone fibres 2 can be folded back through 180 degree and tucked into the weave.

In figure 2 a second DBM scaffold product 7 also comprises a plurality of elongate demineralised bone fibres 8 mechanically interconnected with one another in a regular and repeating pattern. This time the bone fibres 8 are knitted together in a regular and repeating pattern in accordance with the present invention. In particular the bone fibres 8 are arranged in six rows 9, and each bone fibre 8 is formed into loops 10 which pass around the bases 11 of the loops 10 in the row 9 above. In this manner the rows 9 are entwined together into stitches 12 in order to connect them together and to form a cohesive material sheet.

As with the first DBM scaffold product 1 second DBM scaffold product 7 is not likely to have any real application in a clinical sense, as it is too small. However, it is presented in order to illustrate the manner in which the knitted version of the invention can be performed.

In first and second DBM scaffold products 1 and 7 individual filaments 3 of bone are used, but in order to make larger woven DBM scaffold products, and to make any practical knitted DBM products, the bone fibres need to be longer.

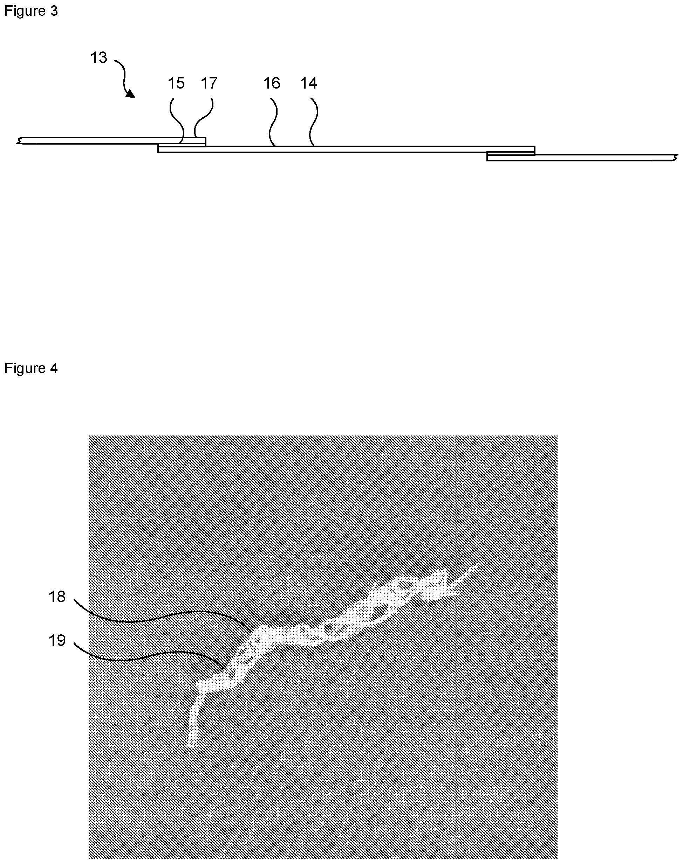

Therefore, Figure 3 shows a section of a composite DBM fibre strip 13 for use in manufacturing a third DBM scaffold product (not shown). The composite strip 13 is made up of a plurality of filaments 14 of bone fibre which are connected together by an adhesive 15. The filaments 14 have flat sides 16, and they are arranged with end portions 17 thereof overlapping, and with the adhesive 15 located between the overlapped end portions 17. The adhesive 15 used in this illustrative example is fibrin glue.

Composite strips like composite strip 13 can be made any length by simply repeating the connections. In this way long composite strips can be formed as strands suitable for weaving or knitting much larger DBM scaffold products than first DBM scaffold product 1 and second DBM scaffold product 7 described above. The same regular and repeating patterns are used, but simply on a larger scale.

In particular, Figure 4 illustrates a fourth DBM scaffold product 18 made using composite strips 19 like composite strip 13. In this case fourth DBM scaffold product 18 was made using a basic single row crocheting method to produce an elongate shape in accordance with the present invention. The regular and repeating pattern therefore only extends along one axis. It will be appreciated that this method can be continued to make DBM scaffold products like this of any length.

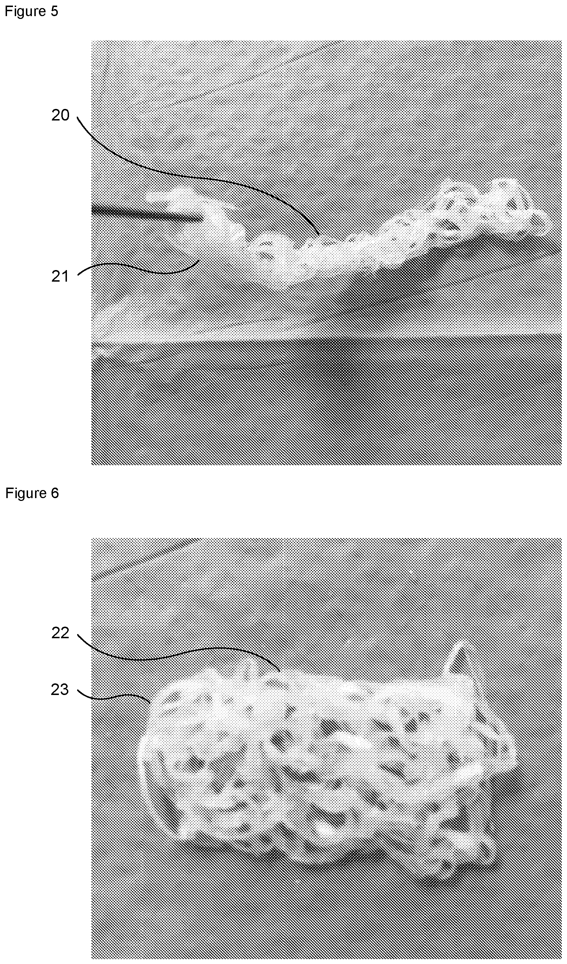

Figure 5 illustrates a fifth DBM scaffold product 20 also made using composite strips 21 like composite strip 13. In this case fifth DBM scaffold product 20 was made using a basic multiple row crocheting method to produce a sheet in accordance with the present invention, which has a regular and repeating pattern which extends along two axes. It will be appreciated that this method can be continued along either axis to make DBM scaffold products of any length and width.

Figure 6 illustrates a sixth DBM scaffold product 22 also made using composite strips 23 like composite strip 13. In this case sixth DBM scaffold product 22 was also made using a basic crocheting method to produce a larger cohesive mass in accordance with the present invention, which has a regular and repeating pattern which extends along two axes.

In the photographs shown in Figures 4 to 6 the regular and repeating pattern of the knitting technique used is not clearly visible due to the flexible nature of the composite strips 19, 21 and 23. In particular, the fourth, fifth and sixth DBM scaffold products 18, 20 and 22 are generally resilient and maliable, and do collapse and distort in shape. However, in each case the particular mechanical interconnection between the composite strips 19, 21 and 23 remains at all times. Namely, the manner in which the composite strips 19, 21 and 23 intertwine between one another in rows of stitches is always maintained.

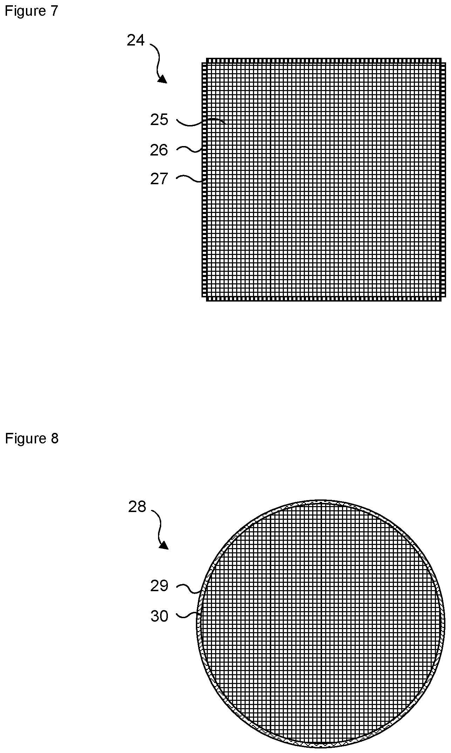

Figure 7 shows a seventh DBM scaffold product 24 which is made using the same weaving technique as employed in the first DBM scaffold product 1, but using composite strips 25 like composite strip 13. As such, it is larger in size. It also has seams 26 at the edges 27 formed by the composite strips 25 being woven back into the weave. The seventh DBM scaffold product 24 has been formed as a square sheet for clinical applications which would require such an application of DBM fibres. The seventh DBM scaffold product 24 can be applied as a sheet, for example to the surface of a bone, or it can be rolled or folded up into suitable other three dimensional shapes for application in any inter bone location where bone regrowth is required. A particular advantage which can be achieved is to orientate the seventh DBM scaffold product 24 with the warp strands thereof bridging an inter bone gap, and the weft strands providing substantially equally spaced keying points for bone re-growth along that gap.

Figure 8 shows an eighth DBM scaffold product 28 which is like the seventh DBM scaffold product 24 described above except it is formed into a circular shape, which may find particular application in certain other clinical procedures. Once again, it has a seam 29 along its edge 30 to prevent the composite strips from becoming detached. The eighth DBM scaffold product 28 can also be applied as a sheet, or it can be rolled or folded up for application.

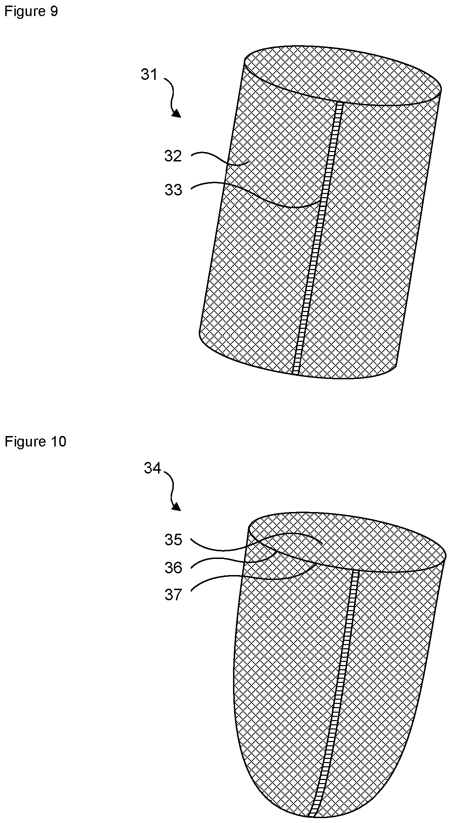

Figure 9 shows a ninth DBM scaffold product 31 which has been made in accordance with the present invention using the same knitting technique as employed in the second DBM scaffold product 7, but using composite strips 32 like composite strip 13. It was created as a sheet, and then closed to make a three dimensional cylinder shape, by means of a knitted seam 33. The ninth DBM scaffold product 31 may find particular application in clinical procedures in which a bone needs to be wrapped in DBM fibres as a surgical technique. The ninth DBM scaffold product 31 can be used a sleeve and placed over such bone.

Figure 10 shows a tenth DBM scaffold product 34 which is like the ninth DBM scaffold product 31 described above but it has been formed as a pouch defining an inner area 35. The tenth DBM scaffold product 34 may find particularly beneficial application in certain clinical procedures where different kinds of bone material can be advantageously located in different areas. For example, autograft or allograft bone chips (not shown) can be placed in the inner area 35, and the tenth DBM scaffold product 34 located in the body such that the DBM bone fibres are adjacent the surfaces of bone to be treated, while the larger bone chips inside the product 34 are held in the area between the surfaces to be treated. This is applicable to filling any large bone defects where DBM alone is not sufficient to fill the volume, and is therefore supplemented by the larger bone chips. It can also be used in posterolateral spinal fusion surgery, where there are bone grafts on both sides of the spine and it is known to use bags of bone chips, which are placed specifically to fill the bone defects. However, unlike in the known products where the bags are simply made of biodegradable resorbable mesh, the tenth DBM scaffold product 34 is made from DBM bone fibres which would significantly improve the performance.

If desired the tenth DBM scaffold product 34 can be closed at the top 36 in order to fully enclose the inner area 35. It is simply a question of using a knitting or seam-making technique to attach one side of the rim 37 to the other. This would capture any autograft or allograft bone chips inside, which may be beneficial for handling prior to implantation, and for when placed inside the body, as the bone chips will not escape.

In the fourth to tenth DBM scaffold products 18, 20, 22, 24, 28, 31 and 34 described above all the composite strips 19, 21, 23, 25, 32 are like composite strip 13, which is made up of bone fibres. However, DBM scaffold products like the fourth to tenth DBM scaffold products 18, 20, 22, 24, 28, 31 and 34 described above can also be made using a combination of such composite strips 13 and elongate synthetic fibres mechanically interconnected together in the same kinds of regular and repeating patterns to make the same kinds of shapes of DBM scaffold product.

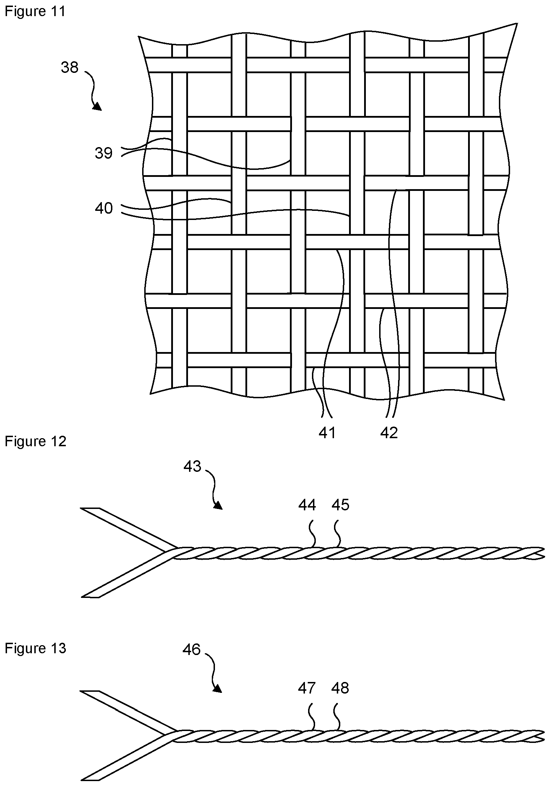

Figure 11 shows a section of an eleventh DBM scaffold product 38 which is like this. Namely, alternate warp strands 39 are like composite strip 13, while the intervening warp strands 40 are made from synthetic fibre strips. Likewise, alternate weft strands 41 are like composite strip 13, while the intervening weft strands 42 are made from synthetic fibre strips. In this illustrative example the synthetic fibre is collagen.

The eleventh DBM scaffold product 38 has a greater tensile strength than the DBM scaffold products described above because the synthetic fibre strips 40, 42 are integrally formed and do not feature any connections secured by the adhesive 15. They therefore provide additional structural support to the composite strips 39 and 41.

In addition to this, the presence of the synthetic fibre strips 40, 42 alters the biodegradation rate of the product 38. It is effectively half that of the fourth to tenth DBM scaffold products 18, 20, 22, 24, 28, 31 and 34 described above as it has half the volume of DBM fibres. This may be preferred for various clinical reasons. It will be appreciated how this can be adjusted by controlling the ratio of the composite strips of DBM fibres to the strips of synthetic fibre. For example, every third warp and weft strand can be a synthetic fibre strip, or every fourth strand and so on.

In the fourth to tenth DBM scaffold products 18, 20, 22, 24, 28, 31 and 34 described above the composite strips 19, 21, 23, 25, 32 are like composite strip 13, which is a simplex strand made up of multiple DBM filaments 15 connected to one another in a line. However, DBM products like the fourth to tenth DBM scaffold products 18, 20, 22, 24, 28, 31 and 34 described above can also be made using more complex composite strips. Figure 12 shows such a wound composite DBM fibre strip 43 for use in manufacturing a twelfth DBM scaffold product (not shown). In this illustrative example two composite strips 44 and 45 are like composite strip 13, but they are wound together to make a simplex yarn or rope-like structure, as shown. This can then be used to make the same kinds of woven or knitted shapes of DBM scaffold product as the fourth to tenth DBM scaffold products 18, 20, 22, 24, 28, 31 and 34 described above. Such DBM scaffold products would have greater tensile strength than the fourth to tenth DBM scaffold products 18, 20, 22, 24, 28, 31 and 34 described above because each wound composite DBM fibre strip 43 is more dense, and because the two composite strips 44 and 45 support each other.

Alternatively DBM products like the fourth to tenth DBM scaffold products 18, 20, 22, 24, 28, 31 and 34 described above can be made using similarly wound strips, but made up of both DBM fibre composite strips like composite strip 13 and synthetic fibre strips. Figure 13 shows such a wound composite strip 46 for use in manufacturing a thirteenth **DBM** scaffold product (not shown). Composite strip 47 is like composite strip 13, and strip 48 is made of a synthetic fibre, and they are wound together to make a simplex yarn or rope-like structure, as shown. **In** this illustrative example the synthetic fibre is chitosan. This wound composite strip 46 can then be used to make the same kinds of woven or knitted shapes of **DBM** scaffold product as the fourth to tenth **DBM** scaffold products 18, 20, 22, 24, 28, 31 and 34 described above. Such products would have greater tensile strength than the fourth to tenth **DBM** scaffold products 18, 20, 22, 24, 28, 31 and 34 described above because each wound composite **DBM** fibre and synthetic fibre strip 46 is more dense, and because the synthetic fibre strip 48 provides extra support to the composite strip 47.

In addition to this, the presence of the synthetic fibre strip 48 alters the biodegradation rate of the end product. It is effectively half that of the fourth to tenth DBM scaffold products 18, 20, 22, 24, 28, 31 and 34 described above as it has half the volume of DBM fibres. This may be preferred for various clinical reasons. It will be appreciated how this can be adjusted by controlling the ratio of DBM fibre strands to synthetic fibre strands, by using both wholly wound composite DBM fibre strips like wound composite DBM fibre strip 43 and mixed DBM fibre and synthetic fibre strips like wound composite DBM fibre and synthetic fibre strip 46. For example, in a woven product every second, third or fourth or so on warp and weft strand can be like wound composite DBM fibre and synthetic fibre strip 46.

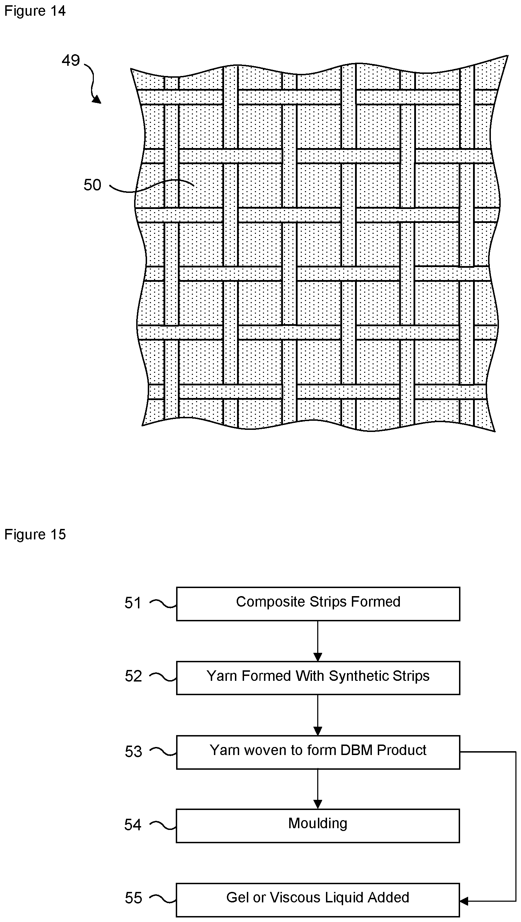

In the fourth to thirteenth DBM scaffold products 18, 20, 22, 24, 28, 31 and 34 described above the composite strips of DBM fibre, wound or otherwise, or the wound composite DBM fibre and synthetic fibre strips are used without any of the known additional viscous compounds usually used with DBM scaffold products. It will be appreciated how it would be possible to add such viscous compounds to such DBM scaffold products. In order to illustrate this Figure 14 shows a sectional front view of a fourteenth DBM scaffold product 49 which is like eleventh DBM scaffold product 38 describe above, but a viscous liquid compound 50 has been added. In this illustrative example the compound is glycerol. Adding the compound 50 makes the fourteenth DBM scaffold product 49 more cohesive as a scaffold and gives it superior handling characteristics.

Figure 15 is a flow diagram illustrating the steps involved in manufacturing a DBM scaffold product.

Namely, in a first step 51 a plurality of elongate demineralised bone fibres are formed, each time by connecting a plurality of filaments taken from donor bones together with adhesive to form a composite strip, like composite strip 13 described above.

In a second step 52 each DBM fibre composite strip is wound together with a synthetic strip made of chitosan, to form yarn or rope-like strips, like wound composite DBM fibre and synthetic fibre strip 46 described above.

In a third step 53 the wound composite strips are woven together in a regular and repeating weave pattern to form a square DBM scaffold product like seventh DBM scaffold product 24 described above. Alternatively, the wound composite strips may be knitted together in a regular and repeating knit pattern in accordance with the present invention.

The method can end there, if a square DBM scaffold product is required for a particular clinical procedure. However, other steps can be carried out to make the DBM product more suitable for use.

For example, in a fourth optional step 54 the DBM scaffold product is placed into a mould of a pre-determined shape and size, before pressure is applied to form the product into a pre-determined shape. For example, a plurality of DBM scaffold sheets can be press fitted together to make more dense sheets. These may find particular application in supporting bone fractures by being wrapped around the fracture site.

Alternatively, the DBM scaffold product is formed into other known implant structures, such as boat like structures which can carry bone chips therein, and which are commonly inserted into the spine in spinal fusion surgery.

In a fifth option step 55, which could be carried out in isolation, or before or after the fourth optional step 54, a gel or viscous liquid is added to the DBM scaffold product in order to make it more cohesive as a scaffold and give it superior handling characteristics.

It will be appreciated that the method illustrated in Figure 15 is merely illustrative, and other steps can be included like those mentioned in detail above. For example, in the third step the DBM product can be formed into a pouch for carrying bone chips. The second step could be omitted so the DBM product was formed only from composite strips of DBM fibres like composite strip 13.

In another alternative embodiment (not shown) DBM scaffold products contain different pre-determined ratios of bone fibres to synthetic fibres than those described above, for example 2:1, 3:1 and so on, depending on the desired biological and physical properties that would be achieved.

Therefore, the present invention provides DBM scaffold products with regular and repeating mechanical interconnection between the fibres. This results in regular performance across the area of the DBM scaffold product because the bone fibres are arranged in the same manner in relation to one another throughout. It also prevents the product from easily deforming or from individual fibres coming loose. It also means that certain two dimensional and three dimensional DBM scaffold product forms can be created by means of the mechanical interconnection method used, which shapes have particular clinical applications. The resulting products are easy to handle, and if flat they can be rolled or folded into more complex shapes depending on the clinical requirement. Depending on the shape and size of the DBM scaffold product it can be used in various surgical procedures such as spinal fusion, bone augmentation, guided tissue regeneration in dental procedures and so on. The product would find particular application in posterolateral spinal fusion where the fibres could be specifically orientated across 2 or 3 lateral spinal processes due to their regular and repeating pattern. This is something which is not possible with any known DBM products in a neat and organised manner. The DBM scaffold product of the present invention is effectively custom made to achieve this.

## Claims

1. A demineralized bone matrix (DBM) scaffold product (7, 18, 20, 22, 31, 34) comprising a plurality of elongate demineralised bone fibres (8) knitted together in a regular and repeating knit pattern.

2. A DBM scaffold product (7, 18, 20, 22, 31, 34) as claimed in claim 1 in which said elongate demineralised bone fibres (8) each comprise a single filament (3) taken from a donor bone.

3. A DBM scaffold product (7, 18, 20, 22, 31, 34) as claimed in claim 1 in which said elongate demineralised bone fibres (8) each comprise a composite strip (13) comprising a plurality of filaments (14) taken from donor bones which are connected together by an adhesive (15).

4. A DBM scaffold product (7, 18, 20, 22, 31, 34) as claimed in claim 3 in which said adhesive (15) comprises a liquid or gel tissue adhesive comprising one or more of a fibrin glue, cyanoacrylate based product, polyethylene glycol-PE-sealant or glutaraldehyde albumin-derived product; said adhesive (15) optionally further comprising a haemostatic component.

5. A DBM scaffold product (7, 18, 20, 22, 31, 34) as claimed in claim 3 in which said filaments (14) comprise flat sides (16), in which said filaments (14) are arranged with end portions (17) thereof overlapping, and with said adhesive (15) located between said overlapped end portions (17).

6. A DBM scaffold product (7, 18, 20, 22, 31, 34) as claimed in claim 1 in which said product is formed into a sheet.

7. A DBM scaffold product (7, 18, 20, 22, 31, 34) as claimed in claim 1 in which said product is formed into a three-dimensional shape defining an inner area.

8. A DBM scaffold product (7, 18, 20, 22, 31, 34) as claimed in claim 1 in which said product comprises a plurality of elongate synthetic fibres (40, 42) mechanically interconnected with said plurality of elongate demineralised bone fibres (8) in a regular and repeating pattern.

9. A DBM scaffold product (7, 18, 20, 22, 31, 34) as claimed in claim 8 in which said synthetic fibres (40, 42) comprises one or more of a polylactic acid, polyglycolic acid, collagen or chitosan.

10. A DBM scaffold product (7, 18, 20, 22, 31, 34) as claimed in claim 1 in which said product further comprises a gel or viscous liquid (50) applied to said plurality of elongate demineralised bone fibres (8).

11. A DBM scaffold product (7, 18, 20, 22, 31, 34) as claimed in claim 10 in which said gel or viscous liquid (50) comprises one or more of glycerol, collagen, gelatin, hyaluronic acid, lecithin, calcium sulphate, poloxamer, calcium phosphate or carboxymethyl cellulose (CMC).

12. A method of manufacturing a DBM scaffold product (7, 18, 20, 22, 31, 34) comprising a plurality of elongate demineralised bone fibres (8), said method comprising the step of knitting said plurality of elongate demineralised bone fibres (8) together with one another in a regular and repeating knit pattern.

13. A method of manufacturing a DBM scaffold product (7, 18, 20, 22, 31, 34) as claimed in claim 12 in which, prior to said step of knitting said plurality of elongate demineralised bone fibres (8) together with one another in a regular and repeating knit pattern, said method comprises the step of forming each of said elongate demineralised bone fibers (8) by connecting a plurality of filaments (14) taken from donor bones together with adhesive (15) to form a composite strip (13), optionally in which said filaments (14) comprise flat sides (16), and said step of forming each of said elongate demineralised bone fibres (8) as a composite strip (13) comprises arranging adjacent filaments (14) with end portions (17) thereof overlapping, and locating said adhesive (15) between said overlapped end portions (17).

14. A method of manufacturing a DBM scaffold product (7, 18, 20, 22, 31, 34) as claimed in claim 12 in which said step of knitting said plurality of elongate demineralised bone fibres (8) together with one another in a regular and repeating knit pattern comprises forming said product as a sheet or forming said product as a three-dimensional shape defining an inner area.

15. A method of manufacturing a DBM scaffold product (7, 18, 20, 22, 31, 34) as claimed in claim 12 in which said method comprises the further step of placing said product into a mould of a pre-determined shape and size and applying a pressure thereto in order to form said product into a pre-determined shape.

## Patentansprüche

1. Gerüstprodukt (7, 18, 20, 22, 31, 34) aus demineralisierter Knochenmatrix (DBM), das eine Vielzahl von länglichen demineralisierten Knochenfasern (8) umfasst, die in einem regelmäßigen und sich wiederholenden Strickmuster zusammengestrickt sind.

2. DBM-Gerüstprodukt (7, 18, 20, 22, 31, 34) nach Anspruch 1, wobei die länglichen demineralisierten Knochenfasern (8) jeweils ein einzelnes Filament (3) umfassen, das einem Spenderknochen entnommen ist.

3. DBM-Gerüstprodukt (7, 18, 20, 22, 31, 34) nach Anspruch 1, wobei die länglichen demineralisierten Knochenfasern (8) jeweils einen Verbundstreifen (13) umfassen, der eine Vielzahl von Filamenten (14) umfasst, die aus Spenderknochen entnommen ist, die durch einen Klebstoff (15) miteinander verbunden sind.

4. DBM-Gerüstprodukt (7, 18, 20, 22, 31, 34) nach Anspruch 3, wobei der Klebstoff (15) einen flüssigen oder gelförmigen Gewebeklebstoff umfasst, der eines oder mehrere von einem Fibrinkleber, Produkt auf Cyanacrylatbasis, Polyethylenglykol-PE-Dichtungsmittel oder aus Glutaraldehydalbumin gewonnenen Produkt umfasst, wobei der Klebstoff (15) optional ferner eine hämostatische Komponente umfasst.

5. DBM-Gerüstprodukt (7, 18, 20, 22, 31, 34) nach Anspruch 3, wobei die Filamente (14) flache Seiten (16) umfassen, wobei die Filamente (14) mit überlappenden Endabschnitten (17) davon angeordnet sind und wobei sich der Klebstoff (15) zwischen den überlappenden Endabschnitten (17) befindet.

6. DBM-Gerüstprodukt (7, 18, 20, 22, 31, 34) nach Anspruch 1, wobei das Produkt zu einer Platte gebildet ist.

7. DBM-Gerüstprodukt (7, 18, 20, 22, 31, 34) nach Anspruch 1, wobei das Produkt zu einer dreidimensionalen Gestalt gebildet ist, die einen Innenbereich definiert.

8. DBM-Gerüstprodukt (7, 18, 20, 22, 31, 34) nach Anspruch 1, wobei das Produkt eine Vielzahl von länglichen synthetischen Fasern (40, 42) umfasst, die mit der Vielzahl von länglichen demineralisierten Knochenfasern (8) in einem regelmäßigen und sich wiederholenden Muster mechanisch verbunden ist.

9. DBM-Gerüstprodukt (7, 18, 20, 22, 31, 34) nach Anspruch 8, wobei die synthetischen Fasern (40, 42) eines oder mehrere von einer Polymilchsäure, Polyglykolsäure, Collagen oder Chitosan umfassen.

10. DBM-Gerüstprodukt (7, 18, 20, 22, 31, 34) nach Anspruch 1, wobei das Produkt ferner ein Gel oder eine viskose Flüssigkeit (50) umfasst, das/die auf die Vielzahl von länglichen demineralisierten Knochenfasern (8) aufgetragen ist.

11. DBM-Gerüstprodukt (7, 18, 20, 22, 31, 34) nach Anspruch 10, wobei das Gel oder die viskose Flüssigkeit (50) eines oder mehrere von Glycerin, Collagen, Gelatine, Hyaluronsäure, Lecithin, Calciumsulfat, Poloxamer, Calciumphosphat oder Carboxymethylcellulose (CMC) umfasst.

12. Verfahren zum Herstellen eines DBM-Gerüstprodukts (7, 18, 20, 22, 31, 34), das eine Vielzahl von länglichen demineralisierten Knochenfasern (8) umfasst, wobei das Verfahren den Schritt des Zusammenstrickens der Vielzahl von länglichen demineralisierten Knochenfasern (8) in einem regelmäßigen und sich wiederholenden Strickmuster umfasst.

13. Verfahren zum Herstellen eines DBM-Gerüstprodukts (7, 18, 20, 22, 31, 34) nach Anspruch 12, wobei vor dem Schritt des Zusammenstrickens der Vielzahl von länglichen demineralisierten Knochenfasern (8) in einem regelmäßigen und sich wiederholenden Strickmuster das Verfahren den Schritt des Bildens von jeder der länglichen demineralisierten Knochenfasern (8) durch Verbinden einer Vielzahl von Filamenten (14), die Spenderknochen entnommen ist, mit Klebstoff (15) umfasst, um einen Verbundstreifen (13) zu bilden, wobei optional die Filamente (14) flache Seiten (16) umfassen, und der Schritt des Bildens von jeder der länglichen demineralisierten Knochenfasern (8) als Verbundstreifen (13) Anordnen von benachbarten Filamenten (14) mit überlappenden Endabschnitten (17) davon und Aufbringen des Klebstoffes (15) zwischen den überlappenden Endabschnitten (17) umfasst.

14. Verfahren zum Herstellen eines DBM-Gerüstprodukts (7, 18, 20, 22, 31, 34) nach Anspruch 12, wobei der Schritt des Zusammenstrickens der Vielzahl von länglichen demineralisierten Knochenfasern (8) in einem regelmäßigen und sich wiederholenden Strickmuster Bilden des Produkts als Platte oder Bilden des Produkts als dreidimensionale Gestalt, die einen Innenbereich definiert, umfasst.

15. Verfahren zum Herstellen eines DBM-Gerüstprodukts (7, 18, 20, 22, 31, 34) nach Anspruch 12, wobei das Verfahren den weiteren Schritt des Platzierens des Produkts in einer Form mit einer vorbestimmten Gestalt und Größe und Ausübens eines Drucks darauf umfasst, um das Produkt zu einer vorbestimmten Gestalt zu bilden.

## Revendications

1. Produit d'échafaudage de matrice osseuse déminéralisée (DBM) (7, 18, 20, 22, 31, 34) comprenant une pluralité de fibres osseuses déminéralisées allongées (8) tricotées ensemble selon un motif de tricot régulier et répétitif.

2. Produit d'échafaudage DBM (7, 18, 20, 22, 31, 34) selon la revendication 1, lesdites fibres osseuses déminéralisées allongées (8) comprenant chacune un seul filament (3) prélevé sur un os donneur.

3. Produit d'échafaudage DBM (7, 18, 20, 22, 31, 34) selon la revendication 1, lesdites fibres osseuses déminéralisées allongées (8) comprenant chacune une bande composite (13) comprenant une pluralité de filaments (14) prélevés sur des os donneurs qui sont reliés ensemble par un adhésif (15).

4. Produit d'échafaudage DBM (7, 18, 20, 22, 31, 34) selon la revendication 3, ledit adhésif (15) comprenant un adhésif tissulaire liquide ou en gel comprenant un ou plusieurs éléments parmi une colle de fibrine, un produit à base de cyanoacrylate, un produit d'étanchéité polyéthylène glycol-PE ou un produit dérivé de glutaraldéhyde-albumine ; ledit adhésif (15) comprenant éventuellement en outre un composant hémostatique.

5. Produit d'échafaudage DBM (7, 18, 20, 22, 31, 34) selon la revendication 3, lesdits filaments (14) comprenant des côtés plats (16), lesdits filaments (14) étant disposés avec des parties d'extrémité (17) de ceux-ci se chevauchant, et avec ledit adhésif (15) situé entre lesdites parties d'extrémité (17) en chevauchement.

6. Produit d'échafaudage DBM (7, 18, 20, 22, 31, 34) selon la revendication 1, ledit produit étant formé en une feuille.

7. Produit d'échafaudage DBM (7, 18, 20, 22, 31, 34) selon la revendication 1, ledit produit étant formé dans une forme tridimensionnelle définissant une zone interne.

8. Produit d'échafaudage DBM (7, 18, 20, 22, 31, 34) selon la revendication 1, ledit produit comprenant une pluralité de fibres synthétiques allongées (40, 42) mécaniquement interreliées à ladite pluralité de fibres osseuses déminéralisées allongées (8) selon un motif régulier et répétitif.

9. Produit d'échafaudage DBM (7, 18, 20, 22, 31, 34) selon la revendication 8, lesdites fibres synthétiques (40, 42) comprenant un ou plusieurs composé parmi un acide polylactique, un acide polyglycolique, le collagène ou le chitosane.

10. Produit d'échafaudage DBM (7, 18, 20, 22, 31, 34) selon la revendication 1, ledit produit comprenant en outre un gel ou un liquide visqueux (50) appliqué sur ladite pluralité de fibres osseuses déminéralisées allongées (8).

11. Produit d'échafaudage DBM (7, 18, 20, 22, 31, 34) selon la revendication 10, ledit gel ou ledit liquide visqueux (50) comprenant un ou plusieurs composés parmi le glycérol, le collagène, la gélatine, l'acide hyaluronique, la lécithine, le sulfate de calcium, le poloxamère, le phosphate de calcium ou la carboxyméthylcellulose (CMC).

12. Procédé de fabrication d'un produit d'échafaudage DBM (7, 18, 20, 22, 31, 34) comprenant une pluralité de fibres osseuses déminéralisées allongées (8), ledit procédé comprenant l'étape de tricotage de ladite pluralité de fibres osseuses déminéralisées allongées (8) ensemble les unes avec les autres selon un motif de tricot régulier et répétitif.

13. Procédé de fabrication d'un produit d'échafaudage DBM (7, 18, 20, 22, 31, 34) selon la revendication 12, avant ladite étape de tricotage de ladite pluralité de fibres osseuses déminéralisées allongées (8) ensemble les unes avec les autres selon un motif de tricotage régulier et répétitif, ledit procédé comprenant l'étape de formation de chacune desdites fibres osseuses déminéralisées allongées (8) en reliant une pluralité de filaments (14) prélevés sur des os donneurs ensemble avec un adhésif (15) de manière à former une bande composite (13), éventuellement lesdits filaments (14) comprenant des côtés plats (16), et ladite étape de formation de chacune desdites fibres osseuses déminéralisées allongées (8) sous forme de bande composite (13) comprenant l'agencement de filaments adjacents (14) avec des parties d'extrémité (17) de ceux-ci se chevauchant, et la localisation dudit adhésif (15) entre lesdites parties d'extrémité (17) en chevauchement.

14. Procédé de fabrication d'un produit d'échafaudage DBM (7, 18, 20, 22, 31, 34) selon la revendication 12, ladite étape de tricotage de ladite pluralité de fibres osseuses déminéralisées allongées (8) ensemble les unes avec les autres selon un motif de tricotage régulier et répétitif comprenant la formation dudit produit sous la forme d'une feuille ou la formation dudit produit sous la forme d'une forme tridimensionnelle définissant une zone interne.

15. Procédé de fabrication d'un produit d'échafaudage DBM (7, 18, 20, 22, 31, 34) selon la revendication 12, ledit procédé comprenant l'étape supplémentaire de placement dudit produit dans un moule d'une forme et d'une taille prédéfinies et d'application d'une pression sur celui-ci afin de former ledit produit dans une forme prédéfinie.
